# EUROPEAN PATENT APPLICATION

(11) **EP 1 172 124 A2**
(43) Date of publication of application: **16.01.2002**
(21) Application number: 01116606.3
(22) Date of filing: 11.07.2001
(51) Int. Cl.: A61M 39/22

(54) **Three-way valve with self-supporting body for medical use**

(30) Priority: 14.07.2000 IT MO000033
(71) Applicant: Sidam di Azzolini Graziano E C. S.a.s., 41030 San Giacomo Roncole - Mirandola, (Prov. of Modena) (IT)
(72) Inventor: Azzolini, Graziano, 41032 Cavezzo (prov. of Modena) (IT)
(74) Representative: Modiano, Guido, Dr.-Ing.

(57) **Abstract**

A three-way valve with self-supporting body (5) for medical use has at least two ways (2, 3) which are controlled by way of unidirectional flow control elements (6) and a third way (4) which is constantly open.

## Description

The present invention relates to a three-way valve with self-supporting body for medical use.

Preassembled throwaway devices, known as "sets", have long been used in the medical field and usually allow to administer drugs or the like to patients, ensuring the sterility of the assembly.

These devices substantially consist of a plurality of mutually interconnected tubes having appropriate standardized couplings with a male or female taper (Luer taper) at their ends and connecting bottles of drugs to needles which are inserted in the venous or arterial circuits of patients.

Valves, throttling devices, insertion points for syringes and other components are then placed along said tubes as necessary in order to be able to perform the intended treatment with maximum safety and efficiency.

In functional terms, these sets overall operate satisfactorily; however, operators in the field, in practice physicians and nurses, increasingly require easy handling which allows to perform with maximum speed and minimum awkwardness the several operations for connecting one another the tubes and the bottles or the tubes and the needles.

In this context, some sets have tubes and connections which, due to the nature of the treatment to be performed, must have at least three useful lines, all of which must be controlled by means of valves being adapted for example to prevent backflows from the patient or to allow mixing and dosage of a plurality of drugs; especially in these cases, operators need to be able to provide the several connections of the set more quickly and easily and also need to be certain that no accidental and dangerous switching of the connections can occur.

The aim of the present invention is to eliminate the above-noted drawbacks of the prior art by providing a three-way valve with self-supporting body for medical use which allows easy grip and handling and connects one another at least said three ways in a controlled manner and without the need to have mutual connection tubes, significantly simplifying the structure of the so-called throwaway medical sets.

This aim and this and other objects which will become better apparent hereinafter are achieved by a three-way valve with self-supporting body for medical use, characterized in that it has at least two of said ways which are controlled by means of unidirectional flow control elements and a third way which is constantly open.

Further characteristics and advantages of the present invention will become better apparent from the description of a preferred embodiment of a three-way valve with self-supporting body for medical use, illustrated only by way of non-limitative example in the accompanying drawing, wherein:

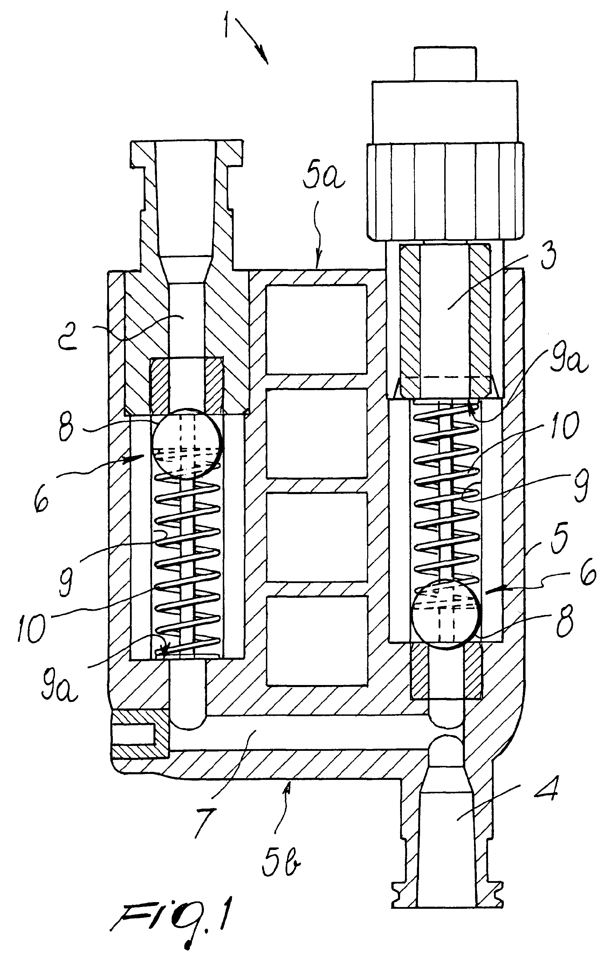

Figure 1 is a sectional view of the valve according to the invention.

With reference to the figure, 1 generally designates a valve with three ways 2, 3, 4 and with a self-supporting body 5 for medical use.

In the valve 1, at least two of said ways, in the specific case the ways 2 and 3, are controlled by means of unidirectional flow control elements 6, and the third way has a constantly open passage 4.

The two ways 2 and 3 are arranged on a common front 5a of the body 5 of the valve 1, while the third way 4 is arranged on the opposite front 5b and is connected to the two ways 2 and 3 by means of an internal duct 7 formed in the body 5.

The two ways 2 and 3 have mutually opposite orientations: the way 2 is an inlet way and the way 3 is a discharge way.

Moreover, the two ways 2 and 3 have mutually opposite couplings, i.e. a first one is female, with a standardized Luer taper, while the second one is male and also has a Luer taper.

Each one of the unidirectional flow control elements 6 is constituted by a ball 8, for closing the corresponding way, such ball 8 being fitted so that it can slide within a hollow seat 9 formed in the body 5 of the valve 1, in contrast with an elastic compression means having a predefined modulus of elasticity, which is substantially constituted, for each ball, by at least one helical spring 10 interposed between the corresponding ball 8 and an abutment bottom 9a of the corresponding hollow seat 9.

The operation of the invention is as follows: the valve 1 is connected by means of connecting tubes, e.g. between a syringe which draws from a bottle containing a drug and a patient into whom said drug is to be infused.

The bottle is connected to the way 2 and the patient is connected to the way 3, while the syringe is connected to the way 4.

The negative pressure generated by the suction of the syringe opens the way 2: the ball 8 compresses the spring 10, and the drug can pass through the way 4, directed towards said syringe.

During the same suction step, the second flow control element of the way 3 is kept firmly closed by the negative pressure produced by suction and by the thrust of the corresponding spring 10, avoiding aspiration from the patient.

Once the syringe has been loaded, it begins to inject the drug towards the patient; flow is thus reversed and the open and closed condition of the flow control elements 6 of the valve 1 is reversed, so that the way 2 is closed while the way 3 opens, because the pressure acting on the ball 8 causes the compression of the corresponding spring 10 and therefore the opening of said way 3, with direct connection between said way and the way 4.

Said way 4, like the ways 2 and 3, is provided with couplings for connection to said tubes; all said couplings form the standard taper known as Luer which is used in the medical field but, for the ways 2 and 3, the couplings are opposite one another, one male and one female, so that operators cannot accidentally perform any dangerous switching of the tubes.

It is also noted that the body 5 of the valve 1 is self-supporting: i.e. it allows health operators to grip it firmly during connection without compromising its functionality; moreover, it allows direct support of the bottles without requiring specifically provided supports, which are inherently awkward and bulky.

In practice it has been found that the described invention achieves the intended aim and objects.

The invention thus conceived is susceptible of numerous modifications and variations, all of which are within the scope of the appended claims.

All the details may further be replaced with other technically equivalent ones.

In practice, the materials used, as well as the shapes and dimensions, may be any according to requirements without abandoning the scope of the protection of the appended claims.

The disclosures in Italian Utility Model Application No. MO2000U000033 from which this application claims priority are incorporated herein by reference.

Where technical features mentioned in any claim are followed by reference signs, those reference signs have been included for the sole purpose of increasing the intelligibility of the claims and accordingly, such reference signs do not have any limiting effect on the interpretation of each element identified by way of example by such reference signs.

## Claims

1. A three-way valve with self-supporting body (5) for medical use, **characterized in that** it has at least two (2, 3) of said ways which are controlled by means of unidirectional flow control elements (6) and a third way (4) which is constantly open.

2. The valve according to claim 1, **characterized in that** said two ways (2, 3) are arranged on a common front (5a) of said valve body (5) and the third way (4) is arranged on the opposite front (5b).

3. The valve according to the preceding claims, **characterized in that** said third way (4) is connected to said two ways (2, 3) by way of an internal duct (7) formed in said valve body (5).

4. The valve according to the preceding claims, **characterized in that** said two ways (2, 3) have mutually opposite orientations, a first one (2) being an inlet way, a second one (3) being a discharge way.

5. The valve according to the preceding claims, **characterized in that** said two ways (2, 3) have mutually opposite couplings, a first coupling (2) being a male coupling with Luer taper and a second coupling (3) being a female coupling which also has a Luer taper.

6. The valve according to the preceding claims, **characterized in that** each one of said unidirectional flow control elements (6) is constituted by a ball (8) for closing a corresponding way, said ball (8) being fitted so that it can slide within a hollow seat (9) formed in said valve body (5) in contrast with an elastic compression means (10) having a predefined modulus of elasticity.

7. The valve according to claim 6, **characterized in that** said elastic compression means is constituted by at least one helical spring (10) which is interposed between the corresponding ball (8) and an abutment bottom (9a) of the corresponding hollow seat (9).

8. The valve according to claim 6, **characterized in that** said hollow seats (9) are made of silicone material.
